# EUROPEAN PATENT SPECIFICATION

(11) **EP 3 541 794 B1**
(45) Date of publication and mention of the grant of the patent: **14.07.2021**
(21) Application number: 17870877.2
(22) Date of filing: 17.11.2017
(51) Int. Cl.: C07D 401/06, A61K 31/4439, A61K 31/4196

(54) **4-((6-(2-(2,4-DIFLUOROPHENYL)-1,1-DIFLUORO-2-HYDROXY-3-(5-MERCAPTO-1H-1,2,4-TRIAZOL-1-YL)PROPYL)PYRIDIN-3-YL)OXY)BENZONITRILE AND PROCESSES OF PREPARATION**
4-((6-(2-(2,4-DIFLUOROPHENYL)-1,1-DIFLUOR-2-HYDROXY-3-(5-MERCAPTO-1H-1,2,4-TRIAZOL-1-YL)PROPYL)PYRIDIN-3-YL)OXY)BENZONITRIL UND VERFAHREN ZUR HERSTELLUNG
PROCÉDÉS DE PRÉPARATION DE 4-((6-(2-(2,4-DUFLUOROPHÉNYL)-1,1-DIFLUORO-2-HYDROXY-3-(5-MERCAPTO-1H-1,2,4-TRIAZOL-1-YL)PROPYL)PYRIDIN-3-YL)OXY)BENZONITRILE

(30) Priority: 18.11.2016 US 201662423876 P
(43) Date of publication of application: 25.09.2019
(73) Proprietor: Dow AgroSciences LLC, Indianapolis, IN 46268 (US)
(72) Inventor: GRAY, Kaitlyn, Indianapolis, IN 46278 (US); YANG, Qiang, Zionsville, IN 46077 (US); BABIJ, Nicholas, R., Indianapolis, IN 46268 (US)
(74) Representative: f & e patent
(86) International application number: PCT/US2017/062132
(87) International publication number: WO 2018/094129

(56) References cited:
- WO-A1-2012/177635
- WO-A1-2015/143188
- WO-A2-2016/187201
- DE-A1- 4 030 039
- US-A1- 2012 088 664
- US-A1- 2013 005 985
- US-A1- 2016 102 072

## Description

### FIELD

Provided herein is 4-((6-(2-(2,4-difluorophenyl)-1,1-difluoro-2-hydroxy-3-(5-mercapto-1*H*-1,2,4-triazol-1-yl)propyl)pyridin-3-yl)oxy)benzonitrile and processes of preparation.

### BACKGROUND

U.S. Patent Application Serial No. 62/163,106 describes *inter alia* certain metalloenzyme inhibitor compounds and their use as fungicides. This patent application describes various routes to generate metalloenzyme inhibiting fungicides. It may be advantageous to provide more direct and efficient methods for the preparation of metalloenzyme inhibiting fungicides and related compounds, *e.g.*, by the use of reagents and/or chemical intermediates which provide improved time and cost efficiency.

### SUMMARY OF THE DISCLOSURE

Provided herein is the compound 4-((6-(2-(2,4-difluorophenyl)-1,1-difluoro-2-hydroxy-3-(5-mercapto-1*H*-1,2,4-triazol-1-yl)propyl)pyridin-3-yl)oxy)benzonitrile (I) and processes for its preparation. In one embodiment, provided herein, is a process for the preparation of the compound of the Formula I: 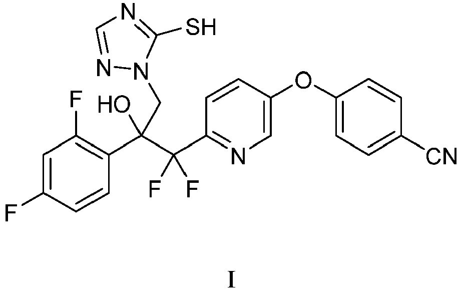 which comprises contacting a compound of Formula II with a base, elemental sulfur and an acid.

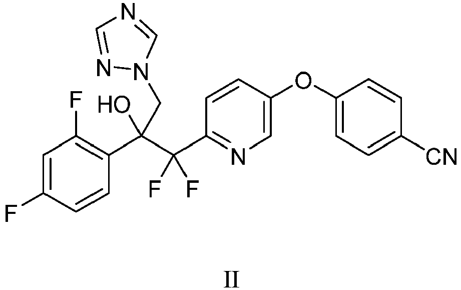

In one embodiment, the compound of Formula II may be prepared by contacting the compound of Formula III 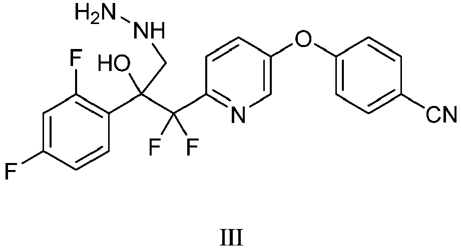 with a nitrogen containing cyclization reagent and optionally an acid.

In another embodiment, the nitrogen containing cyclization reagent for the preparation of the compound of Formula II may consist of 1,3,5-triazine (IV).

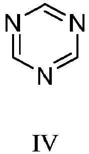

In another embodiment, the nitrogen containing cyclization reagent for the preparation of the compound of Formula II may comprise an *(E)-N-*((((dimethylamino)methylene)-amino)methylene)-*N*-methylmethanaminium halide of the Formula V 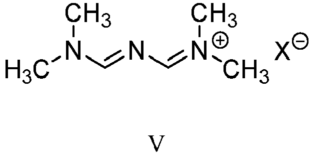 wherein X = Cl or Br.

In another embodiment, the nitrogen containing cyclization reagent for the preparation of the compound of Formula II may comprise *N,N',N*"-methanetriyltriformamide (VI).

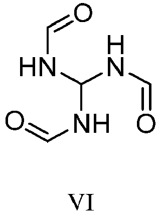

In another embodiment, the nitrogen containing cyclization reagent for the preparation of the compound of Formula II from the compound of Formula III may comprise a formamidine salt of Formula VII, 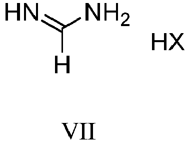 wherein X = OAc, Cl, Br, or I.

In another embodiment, the nitrogen containing cyclization reagent for the preparation of the compound of Formula II from the compound of Formula III may comprise formamide.

The term "halogen" or "halo" refers to one or more halogen atoms, defined as F, Cl, Br, and I. The term "oxirane" refers to a 3-membered ring containing two carbon atoms and one oxygen atom.

The term "organometallic" refers to an organic compound containing a metal, especially a compound in which a metal atom is bonded directly to a carbon atom.

Room temperature (RT) is defined herein as about 20 °C to about 25 °C.

Throughout the disclosure, references to the compounds of Formula I-III are read as also including optical isomers and salts. Specifically, when compounds of Formula I-III contain a chiral carbon, it is understood that such compounds include optical isomers and racemates thereof. Exemplary salts may include: hydrochloride salts, hydrobromide salts, hydroiodide salts, and the like.

Certain compounds disclosed in this document can exist as one or more isomers. It will be appreciated by those skilled in the art that one isomer may be more active than the others. The structures disclosed in the present disclosure are drawn in only one geometric form for clarity, but are intended to represent all geometric and tautomeric forms of the molecule. For example, the chemical structures of Formulas I and Ia are tautomeric forms of the same molecule.

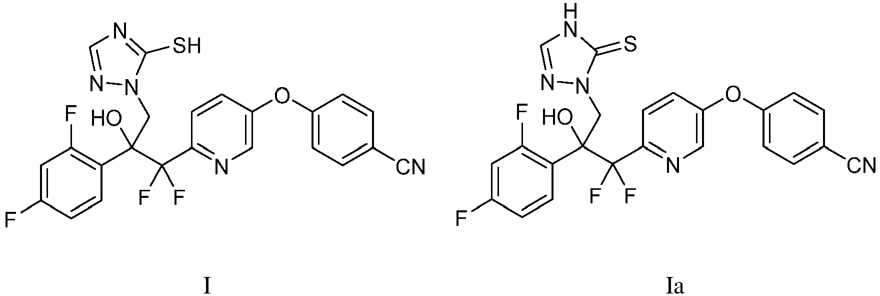

### DETAILED DESCRIPTION

4-((6-(2-(2,4-Difluorophenyl)-1,1-difluoro-2-hydroxy-3-(5-mercapto-1*H*-1,2,4-triazol-1-yl)propyl)pyridin-3-yl)oxy)benzonitrile (I) is provided herein and may be prepared from 4-((6-(2-(2,4-difluorophenyl)-1,1-difluoro-2-hydroxy-3-(1*H*-1,2,4-triazol-1-yl)propyl)pyridin-3-yl)oxy)benzonitrile (II) as shown in Example 1.

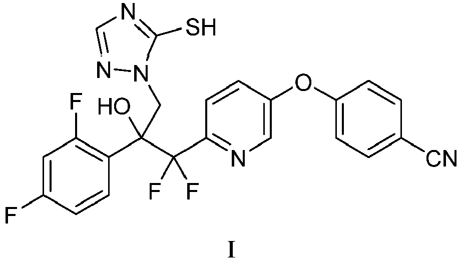

### Example 1: Preparation of 4-((6-(2-(2,4-difluorophenyl)-1,1-difluoro-2-hydroxy-3-(5-mercapto-1H-1,2,4-triazol-1-yl)propyl)pyridin-3-yl)oxy)benzonitrile (I)

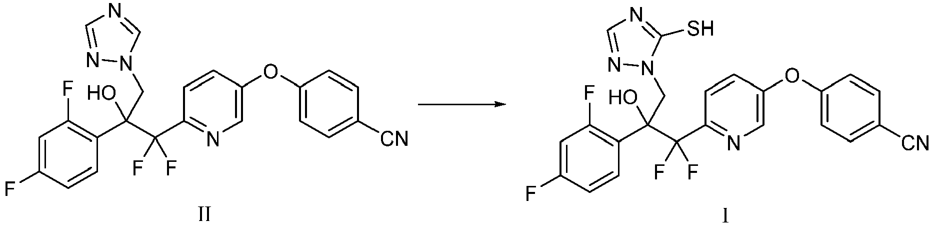

### Method A: Use of lithium bis(trimethylsilyl)amide (LiHMDS) in THF.

To a 1 L three-neck flask equipped with a temperature probe, a nitrogen inlet and a mechanical stirrer was charged 4-((6-(2-(2,4-difluorophenyl)-1,1-difluoro-2-hydroxy-3-(1*H-*1,2,4-triazol-1-yl)propyl)pyridin-3-yl)oxy)benzonitrile (II) (20 g, 42.6 mmol), sulfur (13.66 g, 426 mmol), and THF (85 mL). The reaction mixture was cooled to -40 °C and lithium bis(trimethylsilyl)amide (1.5 M solution in THF, 128 mL, 192 mmol) was added *via* syringe and stirred at -30 °C over 90 min. The reaction was quenched with 4 N HCl and the resulting mixture was stirred for 1 h. The organic layer was washed with brine (2 × 60 mL), and then saturated aqueous sodium bicarbonate (50 mL). The organic layer was filtered and treated with saturated aqueous sodium thiosulfate (200 mL). The resulting mixture was stirred for 1 h and filtered. The organic phase was dried over anhydrous sodium sulfate, filtered, and concentrated to give 21.6 g of a light yellow foam. Acetonitrile (50 mL) was added and the resulting solution was seeded (0.1 g of I) and stirred for 18 h. The suspension was filtered to afford 4.2 g of the title compound. The filtrate was concentrated *in vacuo* to give a light yellow foam. MTBE was added and stirred at 50 °C for 30 min. The suspension was cooled to 20 °C and filtered to afford 11.2 g of the title compound. The MTBE filtrate was concentrated and purified via silica gel column chromatography eluting with ethyl acetate/hexane to afford 2.8 g of the title compound as a white foam. The three lots were combined to afford 18.2 g (85% yield) of 4-((6-(2-(2,4-difluorophenyl)-1,1-difluoro-2-hydroxy-3-(5-mercapto-1*H*-1,2,4-triazol-1-yl)propyl)pyridin-3-yl)oxy)benzonitrile (I). ¹H NMR (400 MHz, DMSO-*d₆*) δ 13.59 (s, 1H), 8.47 (d, *J* = 2.7 Hz, 1H), 8.18 (s, 1H), 8.00-7.85 (m, 2H), 7.71 (dd, *J* = 8.7, 2.8 Hz, 1H), 7.64 (d, *J* = 8.7 Hz, 1H), 7.38 (td, *J* = 9.0, 6.8 Hz, 1H), 7.27 - 7.19 (m, 2H), 7.16 (ddd, *J* = 12.0, 9.1, 2.6 Hz, 1H), 6.96 (td, *J* = 8.5, 2.6 Hz, 1H), 6.42 (s, 1H), 5.26 - 4.82 (m, 2H). ¹⁹F NMR (376 MHz, DMSO-*d*₆) δ -102.20 (td, *J* = 24.5, 8.9 Hz), -108.18 (dd, *J* = 24.8, 17.9 Hz), -111.23 (d, *J* = 8.9 Hz). ESIMS: m/z 502.0 ([M+H]⁺).

### Method B1: Use of lithium bis(trimethylsilyl)amide (LiHMDS) in THF/toluene.

A 500 mL three-neck flask was fitted with a thermocouple, a condenser with a nitrogen inlet, a septum, and a mechanical stirrer. After flushing with nitrogen, 4-((6-(2-(2,4-difluorophenyl)-1,1-difluoro-2-hydroxy-3-(1*H*-1,2,4-triazol-1-yl)propyl)pyridin-3-yl)oxy)benzonitrile (II) (7.5 g, 16.0 mmol) was added followed by dry toluene (100 mL). Sulfur powder (2.3 g, 71.9 mmol) was added. The solution was cooled to -18 to -16 °C and lithium bis(trimethylsilyl)amide (45 mL, 1.0 M solution in THF) was added in portions over 30 min, keeping the temperature of the reaction between -22 to -15 °C. After 2.5 h, additional lithium bis(trimethylsilyl)amide (5 mL, 1.0 M solution in THF) was added and the mixture was stirred for 1 h. Water (100 mL) was added to the reaction mixture keeping the temperature below 16 °C. The mixture was slowly warmed to RT with stirring. The layers were separated, and the aqueous phase was acidified with 2 N HCl (25 mL) to pH 3. The acidified aqueous layer was extracted with ethyl acetate (2 × 60 mL). The organic phase was filtered, and residual water was separated. The organic phase was concentrated to afford a yellow glass. Toluene (150 mL) was added and some of the toluene was removed on the rotovap. The mixture was filtered and concentrated to about 25 mL. The warm solution was seeded and stirred at RT for about 1 h. The suspension was cooled to 5 °C and stirred for 1 h. The solid was collected *via* filtration and washed with toluene. The solid was dried to a constant mass to give 4-((6-(2-(2,4-difluorophenyl)-1,1-difluoro-2-hydroxy-3-(5-mercapto-1*H*-1,2,4-triazol-1-yl)propyl)pyridin-3-yl)oxy)benzonitrile (I) as a yellow solid (6.8 g, 80% yield, 94% purity). Analytical data collected from the yellow solid matched the data obtained from the product prepared by using Method A.

### Method B2: Use of lithium bis(trimethylsilyl)amide (LiHMDS) in THF/toluene.

To a 250 mL jacketed reactor was added 4-((6-(2-(2,4-difluorophenyl)-1,1-difluoro-2-hydroxy-3-(1*H*-1,2,4-triazol-1-yl)propyl)pyridin-3-yl)oxy)benzonitrile (II) (7.5 g, 15.98 mmol), sulfur (5.12 g, 160 mmol), and THF (32 mL) and the mixture was cooled to -30 °C. Lithium bis(trimethylsilyl)amide (LiHMDS, 1.0 M solution in toluene, 80 mL, 80 mmol) was added via syringe pump over 45 min. The reaction was stirred at -30 °C for 30 min then was quenched with 112 mL of water and the jacket warmed to 20 °C. After stirring for 1 h, the layers were separated and the organic layer discarded. The aqueous layer was washed with dichloromethane (75 mL). The aqueous layer was extracted with ethyl acetate (150 mL) and the aqueous layer was discarded. The organic layer was washed with brine (100 mL). Water (75 mL) was added to the organic layer and the pH was adjusted to 5-6 using 2 N HCl. The aqueous layer was discarded. The organic layer was quantified (143.16 g, 5.7 wt%, 8.16 g, 102% in pot yield). The organic layer was atmospherically distilled to -85 mL and was heated to 70 °C. The mixture was seeded and held at 70 °C for 30 min before adding heptane (75 mL) over 10 min. The light slurry was cooled to 20 °C over 10 h. The solids were isolated by filtration and air dried to constant mass giving 4-((6-(2-(2,4-difluorophenyl)-1,1-difluoro-2-hydroxy-3-(5-mercapto-1*H*-1,2,4-triazol-1-yl)propyl)pyridin-3-yl)oxy)benzonitrile (I) as a white solid (6.35 g, 12.41 mmol, 78 % yield). mp 219 - 222 °C; ¹H NMR (400 MHz, DMSO-*d*₆) δ 13.59 (s, 1H), 8.47 (d, *J* = 2.7 Hz, 1H), 8.18 (s, 1H), 8.00 - 7.85 (m, 2H), 7.71 (dd, *J* = 8.7, 2.8 Hz, 1H), 7.64 (d, *J* = 8.7 Hz, 1H), 7.38 (td, *J* = 9.0, 6.8 Hz, 1H), 7.27 - 7.19 (m, 2H), 7.16 (ddd, *J* = 12.0, 9.1, 2.6 Hz, 1H), 6.96 (td, *J* = 8.5, 2.6 Hz, 1H), 6.42 (s, 1H), 5.26 - 4.82 (m, 2H); ¹⁹F NMR (376 MHz, DMSO-*d*₆) δ -101.37 - -104.79 (m), -108.07 (dd, *J* = 23.1, 12.9 Hz), -110.76 (d, *J* = 9.0 Hz); ESIMS: *m*/*z* 502.0 ([M+H]⁺).

### Method B3: Use of lithium bis(trimethylsilyl)amide (LiHMDS) in THF/toluene.

To a jacketed 1 L reactor was charged 4-((6-(2-(2,4-difluorophenyl)-1,1-difluoro-2-hydroxy-3-(1H-1,2,4-triazol-1-yl)propyl)pyridin-3-yl)oxy)benzonitrile (II) (27.96 g, 0.057 mol), followed by sulfur flakes (14.57 g, 0.45 mol). Tetrahydrofuran (THF, 76 g) was added and the reaction was cooled to 2.5 °C. To the mixture was added 1.0 M bis(trimethylsilyl)amide solution in toluene (201.1 g, 0.23 mol) over 2.5 h. Upon completing base addition, the reaction mixture was allowed to warm up to 15 °C. After 30 minutes water (343 g) was added slowly over 20 minutes. The organic layer was discarded and the aqueous layer was extracted with ethyl acetate (252 g). The organic layer was washed with 10 wt% NaCl solution (140 g) followed by 10 wt% aqueous acetic acid solution (140 g). The ethyl acetate was removed by distillation replacing the solvent with 2-propanol (319 g) for crystallization. After cooling the reaction mixture to 58°C, the mixture was seeded with product crystals and stirred at 58°C for 1 h. To the resulting mixture was added water (210 g) at 58 °C over 1 h. The suspension was stirred at 58 °C for 10 h and cooled to 20 °C over 3 h. The crystallized product was isolated by filtration, washed with water (30 g), and air dried to constant mass giving 4-((6-(2-(2,4-difluorophenyl)-1,1-difluoro-2-hydroxy-3-(5-mercapto-1*H*-1,2,4-triazol-1-yl)propyl)pyridin-3-yl)oxy)benzonitrile (25.52 g, 47.9 mmol, 84% yield). Analytical data matched the data obtained from the product prepared by using Method A.

### Method C: Use of lithium diisopropylamide (LDA).

A 100 mL reaction vessel equipped with a temperature probe, a nitrogen inlet and a mechanical stirrer was charged with 4-((6-(2-(2,4-difluorophenyl)-1,1-difluoro-2-hydroxy-3-(1*H*-1,2,4-triazol-1-yl)propyl)pyridin-3-yl)oxy)benzonitrile (II) (1 g, 2.130 mmol), sulfur (301 mg, 9.37 mmol), naphthalene as an internal standard (273 mg, 1 equiv) and THF (16 mL) and the reaction flask was cooled to -20 °C. A separate 25 mL flask equipped with a magnetic stirring bar and nitrogen inlet was backfilled with nitrogen and charged with diisopropylamine (1.314 mL, 9.37 mmol) and THF (5.33 mL). The flask was cooled to 0 °C and *n*-butyllithium (2.5 M in hexanes, 3.75 mL, 9.37 mmol) was added to give a clear, light-yellow homogeneous solution of LDA. The solution of LDA was stirred for 30 min at 0 °C and then was added in increments to the reaction vessel containing II *via* syringe until the starting material had been nearly all consumed (<4% remaining) as judged by HPLC analysis (overall, 2.75 equivalents of LDA were added). The reaction was quenched with water (15 mL) and diluted with ethyl acetate. The layers were separated and the aqueous layer was determined to have a pH of 11. The aqueous layer was lowered to pH 8 with saturated aqueous ammonium chloride and then extracted with ethyl acetate. The aqueous layer was lowered to pH 1 with 2 N HCl and then extracted with ethyl acetate. The combined organic layers were dried over anhydrous sodium sulfate, filtered and concentrated. The crude reaction mixture was dissolved in DCM and purified via silica gel column chromatography eluting with ethyl acetate/hexane to afford the title compound as a yellow foam (640 mg, 57% yield). Analytical data matched the data obtained from the product prepared by using Method A.

Suitable solvents for use in this process step may include tetrahydrofuran (THF), 1,2-dimethoxyethane (DME), diethyl ether, methyl *t*-butyl ether (MTBE), 2-methyltetrahydrofuran (2-Me-THF), toluene, dioxane, and mixtures thereof.

Suitable bases for use in this process step may include lithium bis(trimethylsilyl)amide (LiHMDS), sodium bis(trimethylsilyl)amide (NaHMDS), potassium bis(trimethylsilyl)amide (KHMDS), lithium diisopropylamide (LDA), lithium tetramethylpiperidide (LTMP), and mixtures thereof.

Suitable bases for use in this process step may include lithium bis(trimethylsilyl)amide (LiHMDS), sodium bis(trimethylsilyl)amide (NaHMDS), potassium bis(trimethylsilyl)amide (KHMDS), lithium diisopropylamide (LDA), lithium tetramethylpiperidide (LTMP), metal *t*-butoxides such as lithium *t*-butoxide, sodium t-butoxide, potassium *t*-butoxide, metal carbonates, such as lithium carbonate, sodium carbonate, potassium carbonate, potassium carbonate, and mixtures thereof.

At least about 2 molar equivalents, at least about 3 molar equivalents, or at least about 4 molar equivalents of the base may be used in this process step relative to the compound of Formula II.

Suitable acids for use in this process step may include, but are not limited to, HCl, HBr, H₂SO₄, H₃PO₄, HNO₃, acetic acid and trifluoroacetic acid.

This process step may be conducted at temperatures from about 150 °C to about -80 °C, or from about 100 °C to about -30 °C.

4-((6-(2-(2,4-Difluorophenyl)-1,1-difluoro-2-hydroxy-3-(1*H*-1,2,4-triazol-1-yl)propyl)pyridin-3-yl)oxy)benzonitrile (II) may be prepared from 4-((6-(2-(2,4-difluorophenyl)-1,1-difluoro-3-hydrazino-2-hydroxypropyl)pyridin-3-yl)oxy)benzonitrile (III) as shown in Example 2.

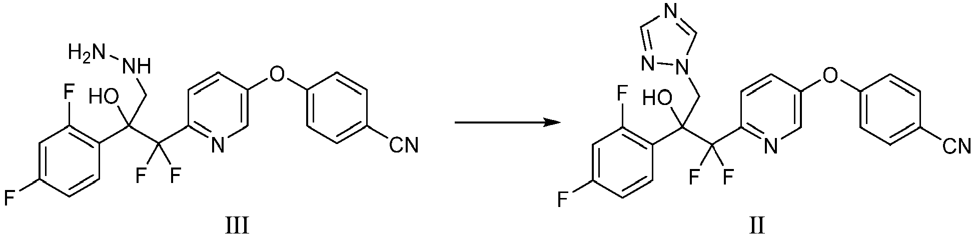

### Example 2: Preparation of 4-((6-(2-(2,4-difluorophenyl)-1,1-difluoro-2-hydroxy-3-(1H-1,2,4-triazol-1-yl)propyl)pyridin-3-yl)oxy)benzonitrile (II).

### Method A: Use of 1,3,5-triazine.

**A1:** To 4-((6-(2-(2,4-difluorophenyl)-1,1-difluoro-3-hydrazino-2-hydroxypropyl)pyridin-3-yl)oxy)benzonitrile (III) (3.5 g, 8.09 mmol) in formic acid (8.38 mL, 219 mmol) was added 1,3,5-triazine (0.656 g, 8.09 mmol). The reaction was stirred at room temperature for 45 min, then 25 wt% NaOH (5 mL) was added using an ice bath to control the exotherm. The resulting solid was dissolved with MTBE (30 mL). Additional 25 wt% NaOH (26 mL) was added making the pH of the aqueous layer 5-6. The layers were separated, and the organic layer was concentrated to an oil, which was allowed to stand overnight to give a solid. MeOH (10 g) was added and the flask heated to 60 °C giving a suspension. Water (4 g) was added, the heat was removed, and the mixture seeded with II. After 2 h, the mixture was concentrated. The methanol was removed by rotary evaporation and ethyl acetate added. The aqueous layer was removed and the organic layer dried over anhydrous sodium sulfate. The organic layer was concentrated to a solid. MeOH (10 g) was added and the slurry was heated to 65 °C giving a clear solution. Water (3.95 g) was added slowly and the clear solution was seeded. The mixture was allowed to cooled to room temperature and stirred overnight. The resulting thick slurry was cooled with an ice bath for 30 min and the solids isolated by filtration. The solid were washed with 3:1 MeOH/water (w/w, 5 mL) and allowed to air dry to constant mass giving 4-((6-(2-(2,4-difluorophenyl)-1,1-difluoro-2-hydroxy-3-(1*H*-1,2,4-triazol-1-yl)propyl)pyridin-3-yl)oxy)benzonitrile (II) as a tan solid (2.74 g, 5.84 mmol, 72.1% yield). ¹H NMR (400 MHz, DMSO-*d*₆) δ 8.47 (d, *J* = 2.7 Hz, 1H), 8.36 (s, 1H), 7.99 - 7.89 (m, 2H), 7.71 (s, 1H), 7.69 (dd, *J* = 8.7, 2.8 Hz, 1H), 7.51 (d, *J* = 8.7 Hz, 1H), 7.30 - 7.19 (m, 3H), 7.13 (ddd, *J* = 12.0, 9.2, 2.6 Hz, 1H), 7.05 (s, 1H), 6.88 (td, *J* = 8.5, 2.6 Hz, 1H), 5.35 (d, *J* = 14.6 Hz, 1H), 4.83 (d, *J* = 14.6 Hz, 1H). ¹⁹F NMR (376 MHz, DMSO-*d*₆) δ -102.83 (td, *J* = 22.5, 21.9, 9.2 Hz), -107.66 (dd, *J* = 21.7, 13.5 Hz), -110.46 (d, *J* = 9.4 Hz). ESIMS *m*/*z* 470.2 [(M+H)⁺].

**A2:** To 4-((6-(2-(2,4-difluorophenyl)-1,1-difluoro-3-hydrazino-2-hydroxypropyl)pyridin-3-yl)oxy)benzonitrile (III) (5 g, 11.56 mmol) in THF (20 mL) was added formic acid (2.218 mL, 57.8 mmol) making a yellow solution. 1,3,5-Triazine (0.703 g, 8.67 mmol) in THF (5 mL) was added at 25 °C causing an exotherm to 35 °C. The reaction was stirred at 25 °C overnight. An additional 200 mg 1,3,5-triazine was added in 1.5 mL THF and the reaction stirred at 25 °C for 1 h. The reaction was quenched with saturated sodium carbonate (20 mL) and the layers separated. The organic layer was concentrated, and the residue was purified by silica gel column chromatography using ethyl acetate/hexanes as the eluent. Product containing fractions were concentrated giving 4-((6-(2-(2,4-difluorophenyl)-1,1-difluoro-2-hydroxy-3-(1*H*-1,2,4-triazol-1-yl)propyl)pyridin-3-yl)oxy)benzonitrile (II) as a white foam (4.05 g, 8.63 mmol, 74.6% yield). Analytical data was consistent with that of previously obtained sample A1.

This process step may be conducted at temperatures from about 0 °C to about 50 °C, or from about 20 °C to about 40 °C.

Suitable solvents for use in this process step may include formic acid, THF, ethanol, methanol, 2-methyl-THF, dioxane, acetonitrile, and mixtures thereof.

Suitable acids for use in this process step may include formic acid, acetic acid, trifluoroacetic acid, hydrobromic acid, and hydrochloric acid.

### Method B: Use of (E)-N-((((dimethylamino)methylene)amino)methylene)-N-methylmethanaminium chloride (Gold's Reagent).

**B1:** To 4-((6-(2-(2,4-difluorophenyl)-1,1-difluoro-3-hydrazinyl-2-hydroxypropyl)pyridin-3-yl)oxy)benzonitrile (III) (0.2 g, 0.463 mmol) and *(E)-N-*((((dimethylamino)methylene)amino)- methylene)-*N*-methylmethanaminium chloride (Gold's Reagent, 0.091 g, 0.555 mmol) in toluene (3 mL) was added acetic acid (0.199 mL, 3.47 mmol). Upon addition of the acetic acid the starting materials started to dissolve. The reaction was heated at 85 °C for 1 h and cooled to room temperature. The reaction mixture was partitioned between ethyl acetate and saturated sodium bicarbonate. The organic layer was concentrated, and the residue was purified using column chromatography with ethyl acetate/hexanes as the eluent. Product containing fractions were collected and concentrated giving 4-((6-(2-(2,4-difluorophenyl)-1,1-difluoro-2-hydroxy-3-(1*H*-1,2,4-triazol-1-yl)propyl)pyridin-3-yl)oxy)benzonitrile (II) as a pale yellow foam (97 mg, 0.203 mmol, 43.8% yield). Spectral data matched that described in Method A.

**B2:** To 4-((6-(2-(2,4-difluorophenyl)-1,1-difluoro-3-hydrazino-2-hydroxypropyl)pyridin-3-yl)oxy)benzonitrile (III) (0.1 g, 0.231 mmol) in THF (1 mL) was added *(E)-N-*((((dimethylamino)methylene)amino)methylene)-*N*-methylmethanaminium chloride (Gold's Reagent, 0.045 g, 0.278 mmol) and the reaction was heated at 60 °C for 17 h. The reaction was quantified by HPLC using naphthalene as an internal standard (48 mg, 0.102 mmol, 44% yield of II).

**B3:** To 4-((6-(2-(2,4-difluorophenyl)-1,1-difluoro-3-hydrazino-2-hydroxypropyl)pyridin-3-yl)oxy)benzonitrile (III) (0.1 g, 0.231 mmol) in ethanol (1 mL) was added *(E)-N-*((((dimethylamino)methylene)amino)methylene)-*N*-methylmethanaminium chloride (Gold's Reagent, 0.045 g, 0.278 mmol) and the reaction was heated at 60 °C for 17 h. The reaction was quantified by HPLC using naphthalene as an internal standard (64.3 mg, 0.137 mmol, 59% yield of II).

**B4:** To 4-((6-(2-(2,4-difluorophenyl)-1,1-difluoro-3-hydrazino-2-hydroxypropyl)pyridin-3-yl)oxy)benzonitrile (III) (0.1 g, 0.231 mmol) in acetonitrile (1 mL) was added *(E)-N-*((((dimethylamino)methylene)amino)methylene)-*N*-methylmethanaminium chloride (Gold's Reagent, 0.045 g, 0.278 mmol) and the reaction was heated at 60 °C for 17 h. The reaction was quantified by HPLC using naphthalene as an internal standard (58.9 mg, 0.125 mmol, 54% yield of II).

(*E*)-*N*-((((dimethylamino)methylene)amino)methylene)-*N*-methylmethanaminium halides of the Formula V, wherein X = Cl or Br, may be utilized in this process.

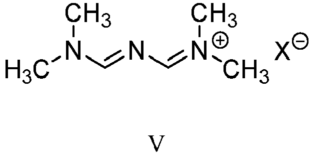

Suitable solvents for use in this process step may include toluene, one or more xylene, THF, ethanol, methanol, acetonitrile, 2-methyl-THF, dioxane, and mixtures thereof.

Suitable acids for use in this process step may include formic acid, acetic acid, trifluoroacetic acid, hydrobromic acid, and hydrochloric acid.

This process step may be conducted at temperatures from about 25 °C to about 150 °C, or from about 50 °C to about 100 °C.

### Method C: Use of N,N,N"-Methanetriyltriformamide.

To 4-((6-(2-(2,4-difluorophenyl)-1,1-difluoro-3-hydrazino-2-hydroxypropyl)pyridin-3-yl)oxy)benzonitrile (III) (0.1 g, 0.231 mmol) in THF (1 mL) was added *N*,*N*,*N*"-methanetriyltriformamide (0.050 g, 0.347 mmol) and HCl (4 M in dioxane, 0.058 mL, 0.231 mmol). The reaction was stirred at room temperature for 1 h and then at 60 °C for 8 h. Naphthalene (14.6 mg) was added as an internal standard for HPLC quantitation (54.2 mg, 50% in pot yield of II).

Suitable solvents for use in this process step may include THF, ethanol, methanol, 2-methyl-THF, dioxane, 1,2-dimethoxyethane (DME) and mixtures thereof.

Suitable acids for use in this process step may include formic acid, acetic acid, trifluoroacetic acid, hydrobromic acid, and hydrochloric acid.

This process step may be conducted at temperatures from about 25 °C to about 125 °C, or from about 25 °C to about 75 °C.

### Method D: Use of Formamidine Acetate.

**D1:** To 4-((6-(2-(2,4-difluorophenyl)-1,1-difluoro-3-hydrazino-2-hydroxypropyl)pyridin-3-yl)oxy)benzonitrile (III) (0.1 g, 0.231 mmol) in acetonitrile (0.5 mL) and MeOH (0.5 mL) was added formamidine acetate (0.036 g, 0.347 mmol). The reaction was stirred at room temperature for 2 h and then at 60 °C for 3 h. Naphthalene (14.1 mg) was added as an internal standard for HPLC quantitation (56.5 mg, 52% in pot yield of II).

**D2:** To 4-((6-(2-(2,4-difluorophenyl)-1,1-difluoro-3-hydrazinyl-2-hydroxypropyl)pyridin-3-yl)oxy)benzonitrile (III) (3 g, 6.94 mmol) in ethanol (14.99 mL) was added formamidine acetate (1.806 g, 17.35 mmol) and the reaction was heated at 80 °C for 1 h. The reaction was allowed to cool to room temperature and diluted with MTBE (30 mL) causing the mixture to cloud. Water (20 mL) was added making the mixture clear and the phases were separated. The organic layer was washed with 20 mL saturated sodium carbonate and the organic layer was concentrated to an oil. The residue was purified by silica gel column chromatography with ethyl acetate/hexanes as the eluent. Product containing fractions were collected and concentrated giving 4-((6-(2-(2,4-difluorophenyl)-1,1-difluoro-2-hydroxy-3-(1*H*-1,2,4-triazol-1-yl)propyl)pyridin-3-yl)oxy)benzonitrile (II) as a white foam (2.6 g, 5.54 mmol, 80% yield). ¹H NMR (400 MHz, DMSO-*d*₆) δ 8.47 (d, *J* = 2.7 Hz, 1H), 8.36 (s, 1H), 7.99 - 7.89 (m, 2H), 7.71 (s, 1H), 7.69 (dd, *J* = 8.7, 2.8 Hz, 1H), 7.51 (d, *J* = 8.7 Hz, 1H), 7.30 - 7.19 (m, 3H), 7.13 (ddd, *J* = 12.0, 9.2, 2.6 Hz, 1H), 7.05 (s, 1H), 6.88 (td, *J* = 8.5, 2.6 Hz, 1H), 5.35 (d, *J* = 14.6 Hz, 1H), 4.83 (d, *J* = 14.6 Hz, 1H). ¹⁹F NMR (376 MHz, DMSO-*d*₆) δ -102.83 (td, *J* = 22.5, 21.9, 9.2 Hz), -107.66 (dd, *J* = 21.7, 13.5 Hz), -110.46 (d, *J* = 9.4 Hz). ESIMS *m*/*z* 470.2 [(M+H)⁺].

Suitable solvents for use in this process step may include acetonitrile, THF, 2-methyl-THF, ethanol, methanol, isopropanol, dioxane, 1,2-dimethoxyethane (DME), and mixtures thereof.

Suitable formamidine salts for use in this process step may include formamidine acetate, formamidine HCl, formamidine HBr, and formamidine HI.

This process step may be conducted at temperatures from about 25 °C to about 150 °C, or from about 25 °C to about 100 °C.

### Method E: Use of Formamide

**E1:** To 4-((6-(2-(2,4-difluorophenyl)-1,1-difluoro-3-hydrazinyl-2-hydroxypropyl)pyridin-3-yl)oxy)benzonitrile (III) (2 g, 4.63 mmol) in formamide (10 mL, 250 mmol) was added HCl (4 M in dioxane, 5.8 mL, 23.1 mmol). The reaction was heated at 160 °C for 7 h. The reaction was allowed to cool to room temperature and was partitioned between ethyl acetate and aqueous sodium bicarbonate. The organic layer was washed with water, dried over sodium sulfate, filtered, and concentrated. The resulting brown oil was purified by column chromatography (0-60% EtOAc/hexanes) giving 4-((6-(2-(2,4-difluorophenyl)-1,1-difluoro-2-hydroxy-3-(1*H*-1,2,4-triazol-1-yl)propyl)pyridin-3-yl)oxy)benzonitrile (II) (1.62 g, 3.45 mmol, 75%) as a tan solid. ¹H NMR (400 MHz, DMSO-*d*₆) δ 8.47 (d, *J* = 2.7 Hz, 1H), 8.36 (s, 1H), 7.99 - 7.89 (m, 2H), 7.71 (s, 1H), 7.69 (dd, *J* = 8.7, 2.8 Hz, 1H), 7.51 (d, *J* = 8.7 Hz, 1H), 7.30 - 7.19 (m, 3H), 7.13 (ddd, *J* = 12.0, 9.2, 2.6 Hz, 1H), 7.05 (s, 1H), 6.88 (td, *J* = 8.5, 2.6 Hz, 1H), 5.35 (d, *J* = 14.6 Hz, 1H), 4.83 (d, *J* = 14.6 Hz, 1H). ¹⁹F NMR (376 MHz, DMSO-*d*₆) δ -102.83 (td, *J* = 22.5, 21.9, 9.2 Hz), -107.66 (dd, *J* = 21.7, 13.5 Hz), -110.46 (d, *J* = 9.4 Hz). ESIMS m/z 470.2 [(M+H)⁺].

**E2:** 4-((6-(2-(2,4-Difluorophenyl)-1,1-difluoro-3-hydrazinyl-2-hydroxypropyl)pyridin-3-yl)oxy)benzonitrile (III) (2 g, 4.63 mmol) in formamide (10 mL, 250 mmol) was heated at 160 °C for 5 h. The reaction was allowed to cool to room temperature and the mixture was diluted with MTBE and ethyl acetate. The resulting mixture was washed with water, dried over sodium sulfate, and filtered. The organic layer was quantified using naphthalene as an HPLC internal standard and indicated the formation of 1.69 g (77%) of 4-((6-(2-(2,4-difluorophenyl)-1,1-difluoro-2-hydroxy-3-(1*H*-1,2,4-triazol-1-yl)propyl)pyridin-3-yl)oxy)benzonitrile (II).

This process step may be conducted in neat formamide or in mixtures of formamide with one or more organic solvents. This process step may include the use of an acid such as for example, hydrochloric acid, or no acid may be used.

This process step may be conducted at temperatures from about 100 °C to about 200 °C, or from about 140 °C to about 180 °C.

### Example 3: Preparation of 4-((6-(2-(2,4-difluorophenyl)-1,1-difluoro-3-hydrazino-2-hydroxypropyl)pyridin-3 -yl)oxy)benzonitrile (III)

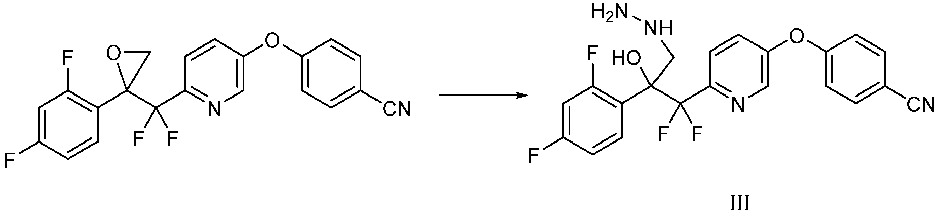

To a slurry of 4-((6-((2-(2,4-difluorophenyl)oxiran-2-yl)difluoromethyl)pyridin-3-yl)oxy)benzonitrile (5 g, 12.49 mmol) in ethanol (50.0 ml) was added anhydrous hydrazine (1.0 ml, 31.2 mmol, 2.5 eq) and the reaction was heated to 60 °C for 4 h, at which point the starting epoxide was completely consumed (monitored by HPLC). The reaction was allowed to cool to room temperature overnight during which time a white precipitate formed. The solids were isolated by filtration and rinsed with ethanol (15 mL) followed by MTBE (15 mL). The solid was dried under vacuum giving 4-((6-(2-(2,4-difluorophenyl)-1,1-difluoro-3-hydrazino-2-hydroxypropyl)pyridin-3-yl)oxy)benzonitrile (III) as an off white solid (4.4 g, about 85% purity, 8.65 mmol, 69.3% corrected yield). ¹H NMR (400 MHz, CDCl₃) δ 8.38 (d, *J* = 2.6 Hz, 1H), 7.70 - 7.65 (m, 2H), 7.64 - 7.55 (m, 1H), 7.45 (d, *J* = 8.6 Hz, 1H), 7.36 (dd, *J* = 8.7, 2.7 Hz, 1H), 7.08 -7.02 (m, 2H), 6.85 - 6.71 (m, 2H), 3.76 (d, *J* = 13.4 Hz, 1H), 3.62 (dd, *J* = 13.4, 2.3 Hz, 1H). ¹⁹F NMR (376 MHz, CDCl₃) δ-105.40 (ddd, *J* = 21.5, 16.2, 8.8 Hz), -109.57 (d, *J* = 21.5 Hz), -109.77 (d, *J* = 16.1 Hz), -110.58 (d, *J* = 8.8 Hz). ESI MS m/z 433.1 [(M+H)⁺].

## Claims

1. A method of making a compound of Formula I comprising: 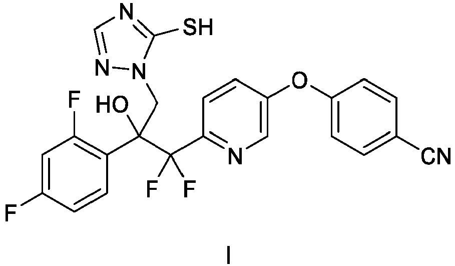 contacting a compound of Formula II 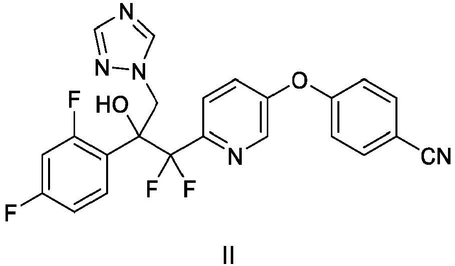 with a base, elemental sulfur and an acid.

2. The method of Claim 1, wherein the base is selected from the group including LiHMDS, NaHMDS, KHMDS, LDA, LTMP and mixtures thereof or from the group including lithium *t*-butoxide, sodium *t*-butoxide, potassium t-butoxide, and mixtures thereof or from the group including lithium carbonate, sodium carbonate, potassium carbonate, cesium carbonate, and mixtures thereof.

3. The method of Claim 1, further comprising a solvent selected from the group including THF, DME, ether, MTBE, 2-Me-THF, dioxane, hexane, toluene, and mixtures thereof.

4. The method of Claim 1, wherein the acid is selected from the group including HCl, HBr, H₂SO₄, H₃PO₄, HNO₃, acetic acid, and trifluoroacetic acid.

5. The method of Claim 1 wherein the contacting is carried out between about 150 °C and about -80 °C or between about 100 °C and about -30 °C.

6. The method of Claim 1, further comprising the step of contacting a compound of Formula III 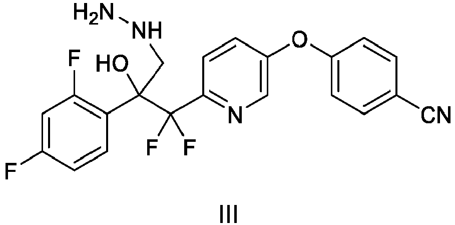 with a nitrogen containing cyclization reagent and optionally an acid, to prepare the compound of Formula II.

7. A method of making a compound of Formula II comprising: 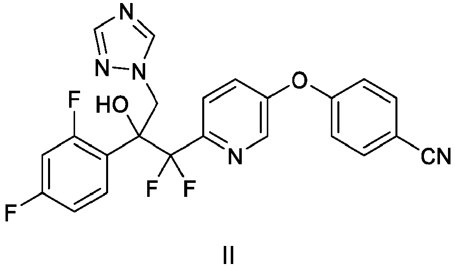 the step of contacting a compound of Formula III 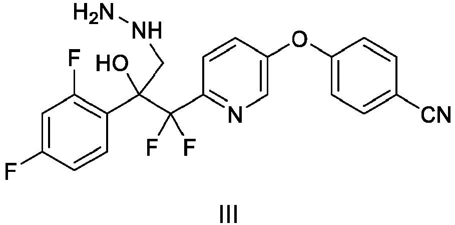 with a nitrogen containing cyclization reagent and optionally an acid.

8. The method of Claims 6-7, wherein the nitrogen containing cyclization reagent comprises 1,3,5-triazine.

9. The method of Claims 6-7, wherein the nitrogen containing cyclization reagent comprises an (*E*)-*N*-((((dimethylamino)methylene)amino)-methylene)-*N*-methylmethanaminium halide of Formula V 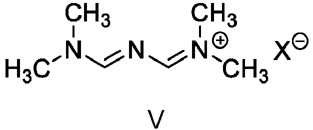 wherein X is Cl or Br.

10. The method of Claims 6-7, wherein the nitrogen containing cyclization reagent comprises *N*,*N'*,*N"*-methanetriyltriformamide of Formula VI.

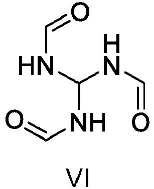

11. The method of Claims 6-7, wherein the nitrogen containing cyclization reagent comprises a formamidine salt of Formula VII 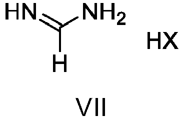 wherein X = OAc, Cl, Br, or I.

12. The method of Claims 6-11, wherein the optional acid may be selected from the group including HCl, formic acid, acetic acid, and trifluoroacetic acid.

13. The method of Claims 6-11 wherein the contacting is carried out between about 50 °C and about 200 °C or between about 50 °C and about 100 °C or between about 20 °C and about 60 °C.

14. The method of Claims 6-7, wherein the nitrogen containing cyclization reagent comprises formamide.

15. The method of Claim 14 wherein the contacting is carried out between about 100 °C and about 200 °C or between about 140 °C and about 180 °C.

## Patentansprüche

1. Ein Verfahren zur Herstellung einer Verbindung der Formel I umfassend 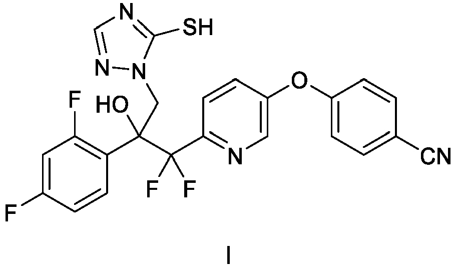 In-Kontakt-Bringen einer Verbindung der Formel II 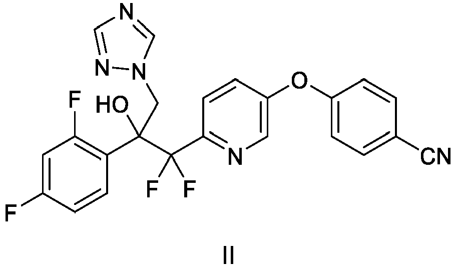 mit einer Base, elementarem Schwefel und einer Säure.

2. Das Verfahren gemäß Anspruch 1, wobei die Base aus der Gruppe enthaltend LiHMDS, NaHMDS, KHMDS, LDA, LTMP und Mischungen davon oder aus der Gruppe enthaltend Lithium-*t*-butoxid, Natrium-t-butoxid, Kalium-*t*-butoxid und Mischungen davon oder aus der Gruppe enthaltend Lithiumcarbonat, Natriumcarbonat, Kaliumcarbonat, Cäsiumcarbonat und Mischungen davon ausgewählt ist.

3. Das Verfahren gemäß Anspruch 1, des Weiteren umfassend ein Lösungsmittel ausgewählt aus der Gruppe enthaltend THF, DME, Ether, MTBE, 2-Me-THF, Dioxan, Hexan, Toluol und Mischungen davon.

4. Das Verfahren gemäß Anspruch 1, wobei die Säure aus der Gruppe enthaltend HCl, HBr, H₂SO₄, H₃PO₄, HNO₃, Essigsäure und Trifluoressigsäure ausgewählt ist.

5. Das Verfahren gemäß Anspruch 1, wobei In-Kontakt-Bringen zwischen etwa 150 °C und etwa -80 °C oder zwischen etwa 100 °C und etwa -30 °C ausgeführt wird.

6. Das Verfahren gemäß Anspruch 1, des Weiteren umfassend den Schritt des In-Kontakt-Bringens einer Verbindung der Formel III 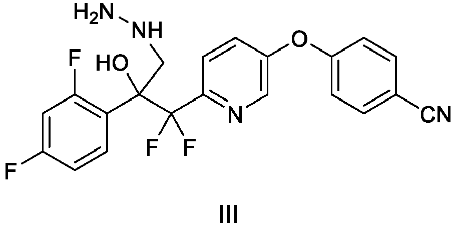 mit einem stickstoffhaltigen Cyclisierungsreagenz und wahlweise einer Säure, um eine Verbindung der Formel II herzustellen.

7. Ein Verfahren zur Herstellung einer Verbindung der Formel II 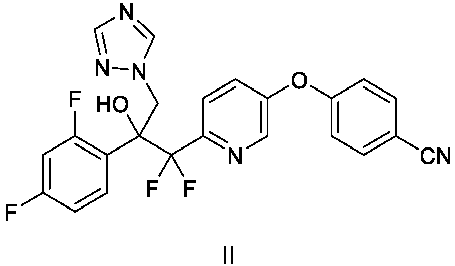 umfassend den Schritt des In-Kontakt-Bringens einer Verbindung der Formel III 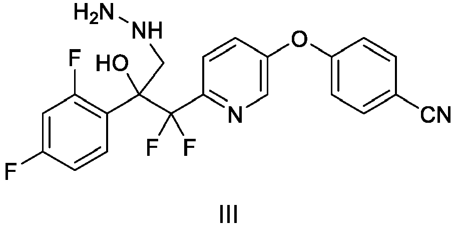 mit einem stickstoffhaltigen Cyclisierungsreagenz und wahlweise einer Säure.

8. Das Verfahren gemäß den Ansprüchen 6 bis 7, wobei das stickstoffhaltige Cyclisierungsreagenz 1,3,5-Triazin umfasst.

9. Das Verfahren gemäß den Ansprüchen 6 bis 7, wobei das stickstoffhaltige Cyclisierungsreagenz ein (*E*)-*N*-((((Dimethylamino)methylen)amino)-methylen)-*N*-methylmethanaminiumhalogenid der Formel V umfasst, 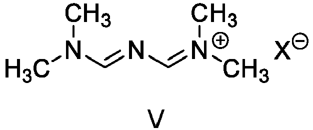 wobei X Cl oder Br ist.

10. Das Verfahren gemäß den Ansprüchen 6 bis 7, wobei das stickstoffhaltige Cyclisierungsreagenz *N*,*N*',*N*"-Methantriyltriformamid der Formel VI umfasst

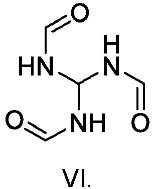

11. Das Verfahren gemäß den Ansprüchen 6 bis 7, wobei das stickstoffhaltige Cyclisierungsreagenz ein Formamidinsalz der Formel VII umfasst, 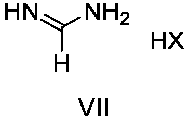 wobei X = OAc, Cl, Br oder I ist.

12. Das Verfahren gemäß den Ansprüchen 6 bis 11, wobei die optionale Säure aus der Gruppe enthaltend HCl, Ameisensäure, Essigsäure und Trifluoressigsäure ausgewählt sein kann.

13. Das Verfahren gemäß den Ansprüchen 6 bis 11, wobei das In-Kontakt-Bringen zwischen etwa 50 °C und etwa 200 °C oder zwischen etwa 50 °C und etwa 100 °C oder zwischen etwa 20 °C und etwa 60 °C ausgeführt wird.

14. Das Verfahren gemäß den Ansprüchen 6 bis 7, wobei das stickstoffhaltige Cyclisierungsreagenz Formamid umfasst.

15. Das Verfahren gemäß Anspruch 14, wobei das In-Kontakt-Bringen zwischen etwa 100 °C und etwa 200 °C oder zwischen etwa 140 °C und etwa 180 °C ausgeführt wird.

## Revendications

1. Procédé de production d'un composé de formule (I), 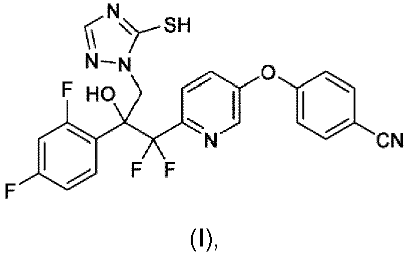 qui comprend le fait de mettre un composé de formule (II), 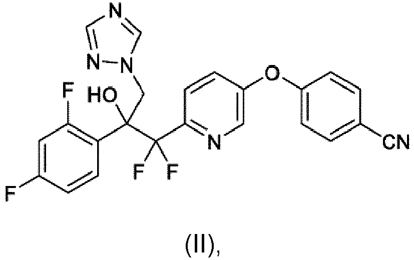 en contact avec une base, du soufre élémentaire et un acide.

2. Procédé selon la revendication 1, dans lequel la base est choisie dans l'ensemble constitué par du bis(triméthylsilyl)-amidure de lithium (LiHMDS), du bis(triméthylsilyl)-amidure de sodium (NaHMDS), du bis(triméthylsilyl)-amidure de potassium (KHMDS), du di-isopropylamidure de lithium (LDA), du tétraméthylpipéridyl-amidure de lithium (LTMP) et des mélanges de ces composés, ou dans l'ensemble constitué par du *t*-butylate de lithium, du *t*-butylate de sodium, du *t*-butylate de potassium et des mélanges de ces composés, ou dans l'ensemble constitué par du carbonate de lithium, du carbonate de sodium, du carbonate de potassium, du carbonate de césium et des mélanges de ces composés.

3. Procédé selon la revendication 1, qui comprend par ailleurs un solvant choisi dans l'ensemble constitué par du tétrahydrofurane (THF), du diméthoxyéthane (DME), de l'éther, de l'éther de méthyle et *t*-butyle (MTBE), du 2-méthyl-tétrahydrofurane (2-Me-THF), du dioxane, de l'hexane, du toluène et des mélanges de ces solvants.

4. Procédé selon la revendication 1, dans lequel l'acide est choisi dans l'ensemble constitué par de l'acide chlorhydrique (HCl), de l'acide bromhydrique (HBr), de l'acide sulfurique (H₂SO₄), de l'acide phosphorique (H₃PO₄), de l'acide nitrique (HNO₃), de l'acide acétique et de l'acide trifluoroacétique.

5. Procédé selon la revendication 1, dans lequel la mise en contact est effectuée à une température comprise dans l'intervalle allant d'environ 150 °C à environ -80 °C ou allant d'environ 100 °C à environ -30 °C.

6. Procédé selon la revendication 1, qui comporte en outre l'étape consistant à mettre un composé de formule (III), 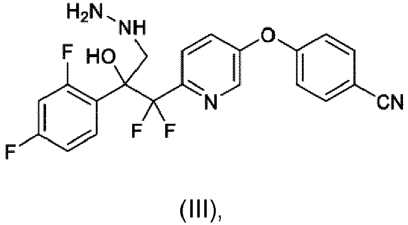 en contact avec un réactif de cyclisation azoté et, en option, un acide pour préparer le composé de formule (II).

7. Procédé de production d'un composé de formule (II), 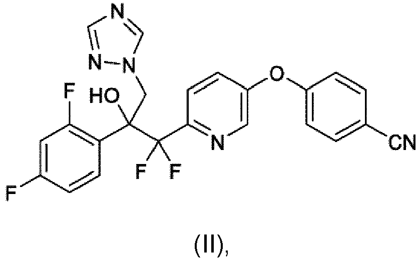 qui comporte l'étape consistant à mettre un composé de formule (III), 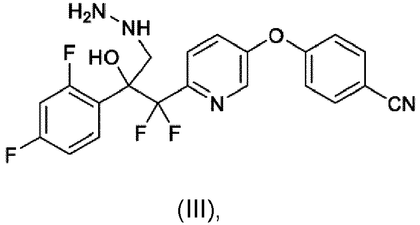 en contact avec un réactif de cyclisation azoté et, en option, un acide.

8. Procédé selon les revendications 6 et 7, dans lequel le réactif de cyclisation azoté comprend de la 1,3,5-triazine.

9. Procédé selon les revendications 6 et 7, dans lequel le réactif de cyclisation azoté comprend un halogénure de (*E*)-*N*-((((diméthylamino)-méthylène)-amino)-méthylène)-*N*-méthyl-méthanaminium répondant à la formule (V), 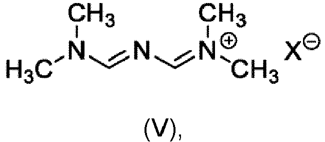 dans laquelle X représente un atome de chlore ou de brome.

10. Procédé selon les revendications 6 et 7, dans lequel le réactif de cyclisation azoté comprend du *N*,*N*',*N*"-méthanetriyl-triformamide, qui répond à la formule (VI),

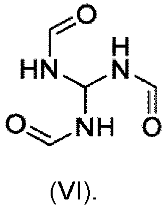

11. Procédé selon les revendications 6 et 7, dans lequel le réactif de cyclisation azoté comprend un sel de formamidine répondant à la formule (VII), 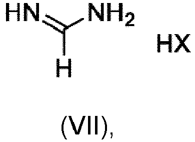 dans laquelle X représente un atome de chlore, de brome ou d'iode, ou un groupe acétate (OAc).

12. Procédé selon les revendications 6 à 11, dans lequel l'acide optionnel peut être choisi dans l'ensemble constitué par de l'acide chlorhydrique (HCl), de l'acide formique, de l'acide acétique et de l'acide trifluoroacétique.

13. Procédé selon les revendications 6 à 11, dans lequel la mise en contact est effectuée à une température comprise dans l'intervalle allant d'environ 50 °C à environ 200 °C, ou allant d'environ 50 °C à environ 100 °C ou allant d'environ 20 °C à environ 60 °C.

14. Procédé selon les revendications 6 et 7, dans lequel le réactif de cyclisation azoté comprend du formamide.

15. Procédé selon la revendication 14, dans lequel la mise en contact est effectuée à une température comprise dans l'intervalle allant d'environ 100 °C à environ 200 °C ou allant d'environ 140 °C à environ 180 °C.
